# FASCICULE DE BREVET EUROPEEN

(11) **EP 0 751 108 B1**
(45) Date de publication et mention de la délivrance du brevet: **12.01.2000**
(21) Numéro de dépôt: 96401151.4
(22) Date de dépôt: 29.05.1996
(51) Int. Cl.: C07C 17/20, C07C 19/08

(54) **Procédé de fabrication du difluorométhane**
Verfahren zur Herstellung von Difluormethan
Process for the manufacture of difluoromethane

(30) Priorité: 29.06.1995 FR 9507821
(43) Date de publication de la demande: 02.01.1997
(73) Titulaire: ELF ATOCHEM S.A., 92800 Puteaux, Hauts-de-Seine (FR)
(72) Inventeur: Requieme, Benoît, 69390 Charly (FR); Perdrieux, Sylvain, 69390 Vernaison (FR); Cheminal, Bernard, 69510 Saucieu-en-Jarrest (FR); Lacroix, Eric, 69480 Amberieux D'Azergues (FR); Lantz, André, 69390 Vernaison (FR)
(74) Mandataire: Leboulenger, Jean

(56) Documents cités:
- WO-A-94/21580
- US-A- 3 183 276
- US-A- 3 855 151
- DATABASE WPI Section Ch, Week 7514 Derwent Publications Ltd., London, GB; Class E16, AN 75-23265W XP002015042 & JP-A-49 134 612 ( ASAHI GLASS CO LTD) , 25 Décembre 1974

## Description

La présente invention concerne le domaine des hydrocarbures fluorés et a plus particulièrement pour objet la fabrication du difluorométhane (F32) par fluoration catalytique du chlorure de méthylène.

Le difluorométhane, connu sous la désignation de F32, est sans danger pour la couche d'ozone. Il est donc particulièrement intéressant pour la substitution des CFC. En mélange avec d'autres hydrofluoroalcanes, tels que le 1,1,1-trifluoroéthane (F143a), le 1,1,1,2-tétrafluoroéthane (F134a) ou le pentafluoroéthane (F125), il est notamment destiné à remplacer le F22 (chlorodifluorométhane) et le F502 (mélange azéotropique de F22 et de chloropentafluoroéthane) dans le domaine de la réfrigération, de l'air conditionné et dans d'autres applications.

Il existe différents procédés connus pour la synthèse du F32. L'hydrogénolyse du F12 (dichlorodifluorométhane) ou du F22 (brevets JP60-01731 et EP 508 660) présente l'inconvénient d'être généralement peu sélective et de sous-produire du méthane non valorisable. Il a récemment été proposé de produire du F32 par fluoration du bis (fluorométhyl) éther (brevet EP 518 506).

Il est également possible de produire du F32 par fluoration du chlorure de méthylène (F30) par de l'HF anhydre. De nombreux brevets décrivent cette réaction, revendiquant l'utilisation de catalyseurs tels que Cr₂O₃, CrF₃, AlF₃, Cr/charbon, Ni/AlF₃...

La difficulté de cette réaction réside dans la stabilité du catalyseur qui a tendance soit à coker rapidement soit à cristalliser. Le problème devient très délicat si l'on veut combiner une forte productivité et une bonne sélectivité tout en maintenant une bonne stabilité du catalyseur.

Pour réduire cette désactivation, il a été proposé l'utilisation de catalyseurs spécifiques tels qu'un mélange mécanique d'alumine et d'oxyde de chrome (brevet GB 821 211). Ce brevet donne un exemple sur la fluoration du chlorure de méthylène mais les productivités en F32 obtenues sur ce catalyseur sont faibles (< 200 g/h/l) et la durée cumulée des tests est inférieure à 5 heures.

Plus généralement, lors des réactions de fluoration, il est très souvent envisagé d'injecter en continu de l'oxygène ou de l'air pour augmenter la durée de vie des catalyseurs. Ainsi le brevet JP 51-82206 revendique l'usage de 0,001 à 1% d'oxygène pour maintenir l'activité de catalyseurs préparés à partir d'oxyde de chrome. Les exemples de ce brevet ne concernent que des réactions de fluoration de molécules perhalogénées (CCl₄, C₂Cl₃F₃).

L'inconvénient majeur de ce procédé est l'apparition d'une réaction de DEACON. En effet, l'oxyde de chrome, bien connu comme catalyseur de fluoration, est également un bon catalyseur d'oxydation de l'HCl (brevets US 4 803 065 et US 4 822 589). L'oxygène introduit au cours de la réaction de fluoration réagit sur l'HCl formé pour produire de l'eau et du chlore. La présence d'eau est, pour des problèmes de corrosion, particulièrement indésirable dans un procédé de fluoration.

L'introduction continue d'une faible quantité de chlore a déjà été proposée dans le brevet JP 49-134612 pour stabiliser l'activité des catalyseurs utilisés pour la dismutation de molécules perhalogénées; dans ce cas, l'utilisation du chlore ne provoque pas de diminution de la sélectivité.

Plus récemment, il a également été décrit l'utilisation de chlore comme inhibiteur de désactivation dans le cas de la fluoration de CF₃CH₂Cl (US Statutory Invention Registration H1129). Les exemples présentés montrent clairement que l'emploi de chlore permet de maintenir une productivité en CF₃CH₂F (F134a) stable. Par contre, aucune indication n'est donnée sur l'effet du chlore sur la sélectivité de la réaction.

Or, dans le cas de molécules hydrogénées et, en particulier dans le cas de la fluoration de CF₃CH₂Cl (F133a), il a été mis en évidence la présence de réactions de chloration, conduisant à la formation de sous-produits non valorisables. Ainsi dans le cas de la fluoration du F133a, il se forme principalement des sous-produits de la série F120 (C₂HClₙF₅₋ₙ).

Etant donné que la réaction de DEACON génère du chlore, cette perte de sélectivité est également observée lors de la fluoration de molécules hydrogénées en présence d'oxygène sur des catalyseurs au chrome. C'est pourquoi, certains brevets (voir par exemple le brevet EP 546 883) ont revendiqué la préparation de catalyseurs spécifiques limitant l'oxydation de l'HCl et la sous production de chlore.

On pouvait supposer que le comportement du chlorure de méthylène serait comparable à celui du F133a, ce qui rendait peu favorable l'utilisation de chlore pour maintenir l'activité du catalyseur. Or, il a été surprenant de constater que, dans le cas de la fluoration du chlorure de méthylène, même avec des teneurs relativement élevées (Cl₂/F30 = 3 % molaire), le chlore réagit très peu sur les produits de la série F30 (CH₂ClₙF₂₋ₙ), ce qui permet son utilisation sans diminution significative de la sélectivité de la réaction.

De plus, dans le brevet JP 51-82206 précité, il est indiqué que l'oxygène permet de maintenir l'activité catalytique même à des teneurs plus basses que celle utilisées avec le chlore. Or, il a été trouvé que, lors de la fluoration du chlorure de méthylène, l'introduction en continu de chlore est, à teneur égale, un moyen plus efficace qu'un apport d'oxygène pour stabiliser l'activité des catalyseurs. En effet, dans des conditions de forte productivité, un apport d'oxygène ne suffit pas à maintenir l'activité des catalyseurs même à température élevée, tandis qu'un apport de chlore permet d'accroître significativement leur durée de vie dés 250°C et donc de réaliser la fluoration du chlorure de méthylène dans un domaine de température où une désactivation irréversible par cristallisation du catalyseur est peu probable.

La présente invention a donc pour objet un procédé de fabrication du difluorométhane par fluoration catalytique en phase gazeuse du chlorure de méthylène au moyen d'HF anhydre, caractérisé en ce que l'on opère en présence de chlore.

Conformément au procédé selon l'invention, on introduit dans le réacteur, du chlore (pur ou dilué dans un gaz inerte tel que l'azote ou l'hélium), en même temps que le chlorure de méthylène et l'HF.

Le rapport molaire Cl₂/CH₂Cl₂ peut varier dans de larges limites et est généralement compris entre 0,01 % et 10 %. On utilise préférentiellement un rapport molaire Cl₂/CH₂Cl₂ compris entre 0,05 et 5 % et, plus particulièrement, un rapport molaire compris entre 0,1 % et 3 %. Il est également possible d'introduire le chlore en le solubilisant dans le chlorure de méthylène.

La température de réaction est généralement comprise entre 200 et 450°C. Cependant, on opère préférentiellement à une température comprise entre 250 et 380°C pour atteindre une productivité importante sans risquer une désactivation du catalyseur par cristallisation.

Les catalyseurs de fluoration à utiliser pour la mise en oeuvre du procédé selon l'invention peuvent être des catalyseurs massiques ou des catalyseurs supportés, le support stable dans le milieu réactionnel étant, par exemple, un charbon actif, une alumine, une alumine partiellement fluorée, le trifluorure d'aluminium ou le phosphate d'aluminium. Par alumine partiellement fluorée, on entend une composition riche en fluor et contenant principalement de l'aluminium, du fluor et de l'oxygène dans des proportions telles que la quantité de fluor exprimée en AlF₃ constitue au moins 50 % du poids total.

Parmi les catalyseurs massiques, on peut citer plus particulièrement l'oxyde de chrome III préparé selon l'une quelconque des méthodes connues de l'homme du métier (procédé sol-gel, précipitation de l'hydroxyde à partir des sels de chrome, réduction de l'anhydride chromique, etc...) et le trifluorure de chrome. Les dérivés de métaux tels que nickel, fer, vanadium (au degré d'oxydation III), manganèse, cobalt, zinc, peuvent également convenir seuls ou en association avec le chrome, sous forme de catalyseurs massiques, mais aussi sous forme de catalyseurs supportés. Il est également possible d'incorporer à ces catalyseurs ou à leur support des alcalino-terreux, des terres rares, du graphite ou de l'alumine pour en accroître la stabilité thermique ou mécanique. Lors de la préparation de catalyseurs associant plusieurs dérivés métalliques, les catalyseurs peuvent être obtenus par mélange mécanique ou par toute autre technique telle qu'une coprécipitation ou une co-imprégnation.

Les catalyseurs supportés ou massiques peuvent être employés sous forme de billes, d'extrudés, de pastilles ou même, si l'on opère en lit fixe, sous forme de morceaux. Lorsque l'on opère en lit fluide, on préfère utiliser un catalyseur sous forme de billes ou d'extrudés.

Comme exemples non limitatifs de catalyseurs, on peut mentionner :
- les microbilles d'oxyde de chrome obtenues par le procédé sol-gel comme décrit dans le brevet FR 2 501 062,
- les catalyseurs d'oxyde de chrome déposé sur charbon actif (brevet US 4 474 895), sur phosphate d'aluminium (brevet EP 55 958) ou sur fluorure d'aluminium (brevets US 4 579 974 et 4 579 976),
- les catalyseurs mixtes d'oxyde de chrome et de chlorure de nickel déposés sur fluorure d'aluminium (demande de brevet EP 0 486 333),
- les catalyseurs massiques à base d'oxyde de chrome cristallisé (demande de brevet EP 657 408),
- les catalyseurs massiques à base d'oxyde de chrome et de nickel (demande de brevet EP 0 546 883),
- les catalyseurs massiques à base d'oxyde de chrome et de vanadium (demande de brevet EP 0 657 409).

Les brevets précités dont le contenu est incorporé ici par référence décrivent largement le mode de préparation de ces catalyseurs, mais aussi leur mode d'activation, c'est-à-dire de transformation préalable du catalyseur en espèces actives stables par fluoration au moyen d'HF gazeux dilué par des composés inertes (azote) ou non (air ou 1,1,2-trichloro-1,2,2-trifluoroéthane). Durant cette activation, les oxydes métalliques servant de matière active (par exemple l'oxyde de chrome) ou de support (par exemple l'alumine) peuvent être partiellement ou complètement transformés en fluorures correspondants.

Sont plus particulièrement préférés les catalyseurs mixtes à base de chrome et de nickel décrits dans les demandes de brevet EP 0 486 333 et EP 0 546 883.

Le temps de contact, défini comme le rapport entre le débit total des réactifs (mesuré dans les conditions de réaction) et le volume de catalyseur, peut varier dans de larges limites et est généralement compris entre 0,01 et 20 secondes. En pratique, on préfère travailler à des temps de contact compris entre 0,1 et 5 secondes.

Cette réaction peut être réalisée à pression atmosphérique ou à une pression supérieure. On choisit préférentiellement une pression comprise entre 1 et 20 bars absolus.

Les exemples suivants illustrent l'invention sans la limiter.

### EXEMPLE 1

### a) Préparation et activation d'un catalyseur à base de nickel et de chrome supportés sur alumine fluorée

Dans un évaporateur rotatif, on place 250 ml d'alumine partiellement fluorée (contenant globalement 83 % en masse de fluorure d'aluminium et 16 % d'alumine), préalablement obtenue par fluoration d'alumine vers 300°C à l'aide d'azote et d'acide fluorhydrique. Ce support fluoré présente avant imprégnation les caractéristiques physico-chimiques suivantes :

| | |
|---|---|
| forme | billes de 1-2 mm de diamètre |
| densité apparente | 0,57 g/ml |
| surface BET | 67 m²/g |
| volume poreux | 0,72 ml/g (pour les pores de rayon compris entre 4 nm et 63 µm) |

Sur le support en agitation on ajoute simultanément une solution aqueuse contenant 12,5 g d'acide chromique CrO₃ et 29 g de chlorure de nickel hexahydraté dans 40 g d'eau, et une solution méthanolique composée de 17,8 g de méthanol dans 50g d'eau. L'imprégnation est effectuée en 45 minutes, à température ambiante et sous pression atmosphérique, sur le support en agitation.

Après séchage sous courant d'azote, en lit fluidisé, vers 110°C pendant 4 heures, le catalyseur est ensuite chargé dans un réacteur en Inconel 600 et activé en lit fixe par un mélange azote/HF suivant la procédure décrite dans le brevet EP 0 486 333. Après ce traitement, les caractéristiques physico-chimiques du catalyseur Ni-Cr/AlF₃ ainsi activé sont les suivantes :

| Composition chimique (pondérale) : | |
|---|---|
| fluor | 58,6 % |
| aluminium | 25,9 % |
| nickel | 6,4 % |
| chrome | 6,0 % |

| Propriétés physiques : | |
|---|---|
| Volume des pores d'un rayon compris entre 4 nm et 63 µm | 0,4 ml/g |
| Surface BET | 23 m²/g |

### b) Fluoration du chlorure de méthylène

Dans un réacteur tubulaire en Inconel 600 de 1 cm de diamètre intérieur et d'un volume de 40 ml, on charge 4 ml de ce catalyseur Ni-Cr/AlF₃, puis on introduit dans un premier temps l'HF et le chlore avec des débits respectifs de 0,45 mole/h et 0,005 mole/h. Le chlorure de méthylène, vaporisé dans un préchauffeur dont la température est fixée à 150°C, est ensuite introduit sous forme gazeuse dans le réacteur à un débit de 0,15 mole/h. La température du réacteur est maintenue à 300°C et le temps de contact dans ces conditions est de 0,5 seconde.

En sortie de réacteur, les produits de réaction sont lavés, séchés et analysés par chromatographie en phase gazeuse. Le tableau suivant résume les résultats obtenus après 48, 171, 338 et 527 heures de fonctionnement continu.

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 48 | 0,5 | 3,1 | 72,1 | 22,5 | 77,1 |
| 171 | 0,5 | 3,0 | 72,2 | 22,6 | 77,3 |
| 338 | 0,5 | 3,2 | 72,0 | 22,5 | 77,4 |
| 527 | 0,4 | 3,4 | 69,6 | 22,9 | 76,8 |

Malgré un apport important de chlore (3 % molaire), les sous-produits de chloration restent minoritaires, dans ces conditions de réaction. Ces sous-produits sont principalement du F20 (trichlorométhane), F21 (fluorodichlorométhane), F22 (chlorodifluorométhane) et F23 (trifluorométhane).

Dans ces conditions de réaction, l'apport de chlore permet de maintenir une activité stable avec une productivité en F32 supérieure à 1100 g/h/l et une sélectivité en F31+F32 supérieure à 99,5 %.

### EXEMPLE 2 (comparatif)

On opère comme à l'exemple 1 sur une charge neuve du même catalyseur Ni-Cr/AlF₃, mais en supprimant l'alimentation en chlore. Les résultats, résumés dans le tableau suivant, montrent qu'en l'absence de chlore le catalyseur se désactive très rapidement.

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 22 | 0,5 | 3,2 | 48,9 | 31,0 | 69,0 |
| 49 | 0,5 | 3,2 | 38,8 | 36,3 | 63,7 |
| 75 | 0,5 | 3,0 | 24,9 | 57,6 | 42,3 |

### EXEMPLE 3 (comparatif)

On opère à nouveau comme à l'exemple 1 sur une charge neuve du même catalyseur Ni-Cr/AlF₃, mais en utilisant de l'oxygène introduit sous forme d'air, à la place du chlore. Le débit d'air est ajusté de manière à ce que le rapport molaire O₂/CH₂Cl₂ soit de 3 %. Les résultats sont rassemblés dans le tableau suivant :

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 24 | 0,5 | 3,1 | 64,2 | 24 | 76 |
| 90 | 0,5 | 3,0 | 46,6 | 35 | 65 |
| 188 | 0,5 | 3,1 | 35,5 | 46 | 54 |

On constate que l'oxygène ne permet pas de maintenir l'activité du catalyseur. A la fin du test de fluoration, le solide est coké; sa teneur pondérale en carbone est de 2,5 %.

### EXEMPLE 4

Le catalyseur est un oxyde de chrome massique ayant une surface spécifique de 209 m²/g et un volume poreux (4 nm<r<63µm) de 0,1 ml/g. Ce solide est utilisé après une activation par l'HF anhydre. Pour cela, l'oxyde de chrome est tout d'abord séché à 200°C puis traité par un mélange N₂/HF à 200°C. Lorsque l'exothermie initiale est passée, on augmente la température jusqu'à 380°C. Le catalyseur est ensuite maintenu à 380°C, sous un débit d'HF anhydre pur, pendant 18 heures.

Le catalyseur activé a les propriétés physico-chimiques suivantes :

| | |
|---|---|
| teneur pondérale en fluor | 27 % |
| teneur pondérale en chrome | 53 % |
| volume des pores d'un rayon compris entre 4 nm et 63 µm | 0,13 ml/g |
| surface BET | 101 m²/g |

La fluoration du chlorure de méthylène est réalisée sur ce catalyseur dans les conditions de l'exemple 1. Après lavage et séchage, l'analyse des produits de réaction par chromatographie en phase gazeuse a donné les résultats regroupés dans le tableau suivant :

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 52 | 0,3 | 3,0 | 66,4 | 24,4 | 73,3 |
| 72 | 0,3 | 2,9 | 66,8 | 24,4 | 75,0 |
| 96 | 0,3 | 2,9 | 67,2 | 23,8 | 73,8 |
| 117 | 0,3 | 2,8 | 66,5 | 24,3 | 73,9 |
| 212 | 0,3 | 2,9 | 65,9 | 24,3 | 73,7 |
| 300 | 0,3 | 3,0 | 68,2 | 23,0 | 73,9 |

Comme dans l'exemple 1 et dans ces conditions de réaction, les produits de chloration restent minoritaires (sélectivité en F31+F32 > 98 %). Les sous-produits sont principalement du F20 (trichlorométhane), F21 (fluorodichlorométhane), F22 (chlorodifluorméthane) et F23 (trifluorométhane) et se forment avec des sélectivités moyennes respectives de 0,5 %, 0,1 %, 0,1 % et 1,3 %.

### EXEMPLE 5

Dans un réacteur tubulaire en Inconel 600 de 21 mm de diamètre intérieur et de 150 ml de volume, on charge 55 ml du catalyseur Ni-Cr/AlF₃ décrit à l'exemple 1-a, puis on alimente, sous une pression de 1,5 MPa (absolus), à 300°C, les réactifs suivants :

| | |
|---|---|
| - HF | à un débit de 3 moles/heure |
| - chlorure de méthylène | à un débit de 1 mole/heure |
| - chlore | à un débit de 0,02 mole/heure |

Le temps de contact sur le catalyseur, dans ces conditions, est de 15 secondes.

En sortie de réacteur, les gaz bruts de réaction sont analysés par chromatographie en phase gazeuse.

Le tableau suivant résume les résultats obtenus après 346 et 376 heures de fonctionnement continu.

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 346 | 15,0 | 3,1 | 66 | 25 | 72 |
| 376 | 15,1 | 3,0 | 65 | 25 | 72 |

Les sous-produits restent minoritaires (série F20).

Dans ces conditions, l'addition de chlore en continu permet de maintenir une activité stable avec une productivité en F32 de 470 g/heure/litre de catalyseur et une sélectivité en F31 + F32 de 97 %.

### EXEMPLE 6 (Comparatif)

Il est réalisé avec la même charge de catalyseur que celle décrite dans l'exemple 5, mais en coupant l'alimentation en chlore pendant 4 heures (autres conditions identiques).

Les résultats obtenus après 383, 385 et 387 heures de fonctionnement continu dans ces conditions figurent dans le tableau ci-dessous.

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 383 | 14,9 | 2,8 | 67 | 27 | 69 |
| 385 | 14,9 | 2,8 | 63 | 29 | 68 |
| 387 | 14,9 | 2,8 | 60 | 30 | 67 |

On constate que l'absence de chlore, même durant quelques heures de fonctionnement, entraîne une baisse notable de l'activité catalytique (environ 13 % en 4 heures), ce qui provoque une baisse de la productivité en F32 de 421 à 366 g/heure/litre de catalyseur.

### EXEMPLE 7

On procède comme dans l'exemple 5, avec le catalyseur Ni-Cr/AlF₃ sous pression (1,5 MPa), mais sur une charge neuve (35 ml) et avec un temps de contact de 5 secondes à 250°C.

Les débits d'alimentation des réactifs sont les suivants :

| | |
|---|---|
| - HF | 6,6 moles/heure |
| - chlorure de méthylène | 2,2 mole/heure |
| - chlore | 0,04 mole/heure |

Le tableau suivant résume les résultats obtenus au bout de 119, 500 et 785 heures de fonctionnement continu dans ces conditions.

| **Durée (h)** | **Temps de contact (s)** | **Rapport molaire HF/F30** | **Conversion du F30 (% molaire)** | **Sélectivité en F31 (% molaire)** | **Sélectivité en F32 (% molaire)** |
|---|---|---|---|---|---|
| 119 | 4,9 | 3,1 | 60,2 | 23,5 | 74,4 |
| 500 | 5,0 | 3,1 | 60,8 | 23,2 | 74,8 |
| 785 | 4,9 | 3,1 | 59,3 | 24,1 | 74,0 |

Malgré une productivité élevée en F32 (1435 g/heure/litre de catalyseur), l'addition de chlore permet de maintenir l'activité catalytique et une sélectivité en F31 + F32 d'environ 98 %.

## Revendications

1. Procédé de fabrication du difluorométhane par fluoration catalytique en phase gazeuse du chlorure de méthylène au moyen d'acide fluorhydrique anhydre, caractérisé en ce que l'on opère en présence de chlore.

2. Procédé selon la revendication 1, dans lequel le rapport molaire Cl₂/CH₂Cl₂ est compris entre 0,01 % et 10 %, de préférence entre 0,05 et 5 % et, plus particulièrement entre 0,1 et 3 %.

3. Procédé selon la revendication 1 ou 2, dans lequel on opère à une température comprise entre 200 et 450°C, de préférence entre 250 et 380°C.

4. Procédé selon l'une des revendications 1 à 3, dans lequel on utilise un catalyseur mixte, massique ou supporté, à base de chrome et de nickel.

5. Procédé selon l'une des revendications 1 à 4, dans lequel le temps de contact est compris entre 0,01 et 20 secondes, de préférence entre 0,1 et 5 secondes

6. Procédé selon l'une des revendications 1 à 6, dans lequel on opère à une pression comprise entre 1 et 20 bars absolus.

## Claims

1. Process for the manufacture of difluoromethane by gas-phase catalytic fluorination of methylene chloride by means of anhydrous hydrofluoric acid, characterized in that the operation is carried out in the presence of chlorine.

2. Process according to Claim 1, in which the Cl₂/CH₂Cl₂ molar ratio is between 0.01% and 10%, preferably between 0.05 and 5% and, more particularly, between 0.1 and 3%.

3. Process according to Claim 1 or 2, in which the operation is carried out at a temperature of between 200 and 450°C, preferably between 250 and 380°C.

4. Process according to one of Claims 1 to 3, in which a solid or supported mixed catalyst based on chromium and nickel is employed.

5. Process according to one of Claims 1 to 4, in which the contact time is between 0.01 and 20 seconds, preferably between 0.1 and 5 seconds.

6. Process according to one of Claims 1 to 6, in which the operation is carried out at a pressure of between 1 and 20 bar absolute.

## Patentansprüche

1. Verfahren zur Herstellung von Difluormethan durch katalytische Fluorierung von Methylenchlorid mit wasserfreier Flußsäure in der Gasphase, dadurch gekennzeichnet, daß man in Gegenwart von Chlor arbeitet.

2. Verfahren nach Anspruch 1, bei dem das Molverhältnis Cl₂/CH₂Cl₂ zwischen 0,01 % und 10 %, vorzugsweise zwischen 0,05 % und 5 % und noch spezieller zwischen 0,1 % und 3 %, liegt.

3. Verfahren nach Anspruch 1 oder 2, bei dem man bei einer Temperatur zwischen 200 und 450 °C, vorzugsweise zwischen 250 und 380 °C, arbeitet.

4. Verfahren nach einem der Ansprüche 1 bis 3, bei dem man einen Misch-, Massen- oder Trägerkatalysator auf Chrom- und Nickelbasis verwendet.

5. Verfahren nach einem der Ansprüche 1 bis 4, bei den, die Kontaktzeit zwischen 0,01 und 20 Sekunden, vorzugsweise zwischen 0,1 und 5 Sekunden, liegt.

6. Verfahren nach einem der Ansprüche 1 bis 6, bei dem man bei einem Absolutdruck zwischen 1 und 20 bar arbeitet.
